# EUROPEAN PATENT APPLICATION

(11) **EP 4 592 312 A1**
(43) Date of publication of application: **30.07.2025**
(21) Application number: 23868578.8
(22) Date of filing: 20.09.2023
(51) Int. Cl.: C07K 16/18, A61P 11/00, G01N 33/68, A61K 39/00

(54) **NOVEL ANTIBODY FOR PREVENTION OR TREATMENT OF FIBROTIC DISEASE**

(30) Priority: 21.09.2022 KR 20220119163
(71) Applicant: INDUSTRY-ACADEMIC COOPERATION FOUNDATION, YONSEI UNIVERSITY, Seoul 03722 (KR)
(72) Inventor: YOON, Hogeun, Goyang-si Gyeonggi-do 10450 (KR); PARK, Soo-Yeon, Seongnam-si Gyeonggi-do 13469 (KR)
(74) Representative: Schnappauf, Georg
(86) International application number: PCT/KR2023/014241
(87) International publication number: WO 2024/063526

(57) **Abstract**

The present invention relates to a method for prevention or treatment of fibrotic disease, wherein an antibody binding specifically to Asporin protein involved in fibrosis is used for predicting the onset of fibrotic disease or for inhibiting the activity of the corresponding protein. The present invention provides an effective antibody that can be used for the diagnosis, prevention, and treatment of idiopathic pulmonary fibrosis, a condition for which efficient treatment methods have been absent because a clear pathogenic mechanism is not known therefor. In addition, the present invention can be advantageously used to effectively block fibrosis in various tissues by ultimately providing a fundamental inhibitory method for pathological fibrosis mechanisms.

## Description

### Technical Field

The present invention relates to methods for predicting the development of fibrotic diseases using antibodies that specifically bind to the Asporin (ASPN) protein involved in fibrosis, or for preventing or treating fibrotic diseases by inhibiting the activity of the protein.

### Background Art

Most organs undergo an inflammatory response and healing process after tissue damage, but in the case of continued damage or irreparable stimuli, the healing process causes tissue to become fibrotic, leading to impaired organ function. In the lungs, one of the most common fibrosis-causing diseases is idiopathic pulmonary fibrosis (IPF), a disease that typically develops between the ages of 50 and 70 and has a poor prognosis, with a survival rate of only 20 to 30 percent after five years. To date, the cause of the disease is not well understood, and only a few treatments exist with very limited effectiveness, including lung transplantation, so there is an urgent need to develop new therapies.

Fibrosis is a reparative or reactive process characterized by the formation and deposition of excessive fibrous connective tissue and is the primary pathogenesis of several fibrotic diseases and chronic conditions. In particular, pulmonary fibrosis is thought to be caused by excessive deposition of extracellular matrix (ECM) and damage to abnormal alveolar epithelial cells, although the exact mechanisms are unknown. Under normal conditions, fibroblasts play an important role in wound repair and connective tissue formation, but pathologic conditions develop when uncontrolled excessive fibroblast expression leads to the accumulation of excessive immune cells and ECM proteins in foci. In addition to these outlined mechanisms, the predictors and pathogenesis of pulmonary fibrosis are still ambiguous, and elucidating the exact molecular and biological mechanisms is crucial for the diagnosis or treatment of pulmonary fibrosis.

TGF-β is upregulated and activated in fibrosis and fibrotic diseases, and is an important signaling mechanism in fibrosis. TGF-β induces an increase in the Extracellular Matrix Gene Set (ECM gene set), which includes matricellular proteins, which are non-structural proteins in the ECM. Rather than functioning as stable structural elements of the ECM, matricellular proteins are pro-fibrotic elements that act as regulators within the tissue to activate fibroblasts. Pamrevlumab, currently in clinical trials for pulmonary fibrosis, is one such targeted therapy that targets CTGF, a member of the matrix cell protein family.

However, treatments for fibrosis, particularly pulmonary fibrosis, that target Asporin proteins, a member of the SLRP (Small Leucine-rich Repeat Proteoglycan) protein family of ECM proteins, are less well understood.

Focusing on the association of the protein Asporin with fibrosis, the inventors sought to develop a monoclonal antibody specific for Asporin as a therapeutic target for fibrosis and to demonstrate the anti-fibrotic effects of the antibody.

Numerous papers and patent references are referenced and cited throughout this specification. The disclosures of the cited papers and patent references are incorporated herein by reference in their entirety to more clearly describe the present invention and the state of the art to which it belongs.

### DISCLOSURE

### Technical Problem

The inventors have been working diligently to discover an efficient treatment for Idiopathic Pulmonary Fibrosis (IPF), an incurable disease with a high prevalence worldwide and no effective treatment options except lung transplantation. As a result, the inventors have identified the role and function of Asporin in the pathogenesis of lung fibrosis and developed a novel monoclonal neutralizing antibody that specifically recognizes and inhibits Asporin to prevent or treat lung fibrosis.

Therefore, an object of the present invention is to provide an antibody to an Asporin protein or an antigen-binding fragment thereof and a nucleic acid molecule encoding the same.

Another object of the present invention is to provide compositions for the prevention or treatment of fibrotic disease.

Still another object of the present invention is to provide diagnostic compositions comprising an antibody of the present invention or an antigen-binding fragment thereof as an active ingredient.

Other objects and advantages of the present invention will become apparent from the detailed description, claims, and drawings of the invention below.

### Technical Solution

According to one aspect of the invention, the present invention provides an antibody or antigen-binding fragment thereof directed against an Asporin protein, comprising a heavy chain variable region having a heavy chain complementarity determining region (CDR) amino acid sequence of HCDR1 having the sequence of SEQ ID NO: 1, HCDR2 having the sequence of SEQ ID NO: 2, and HCDR3 having the sequence of SEQ ID NO: 3.

The inventors have been working diligently to discover an efficient treatment for Idiopathic Pulmonary Fibrosis (IPF), an incurable disease with a high prevalence worldwide and no effective treatment options, except for lung transplantation. As a result, we not only identified the role of Asporin protein in the pathogenesis of lung fibrosis, which causes fibroblast-mediated fibrosis, and confirmed that Asporin protein can be a therapeutic target, but also developed a novel monoclonal neutralizing antibody that recognizes and inhibits Asporin protein with high affinity.

As used herein, the term "antibody" refers to an antibody to an Asporin protein that specifically recognizes and binds to a specific epitope thereof, and includes the complete antibody form as well as antigen-binding fragments of the antibody molecule (antibody fragments).

A complete antibody is a structure with two full-length light chains and two full-length heavy chains, each of which is linked to the heavy chain by a disulfide bond. The heavy chain invariant regions are of the gamma (γ), mu (µ), alpha (α), delta (δ), and epsilon (ε) types with subclasses gamma1 (γ1), gamma2 (γ2), gamma3 (γ3), gamma4 (γ4), alpha1 (α1), and alpha2 (α2). Invariant regions of light chains have kappa (κ) and lambda (λ) types.

As used herein, the term "antigen-binding fragment of an antibody" refers to a fragment within a whole antibody molecule that possesses antigen-antibody binding functionality, and includes Fab, F(ab'), F(ab')2, and Fv. Among the antibody fragments, Fab is a structure with variable regions of the light and heavy chains, an invariant region of the light chain and the first invariant region of the heavy chain (C(H1)), and has one antigen-binding site. Fab' differs from Fab in that it has a hinge region containing one or more cysteine residues at the C-terminus of the heavy chain C(H1) domain. F(ab')2 antibodies are generated when the cysteine residues in the hinge region of Fab' form a disulfide bond. Recombinant techniques for generating Fv fragments with minimal antibody fragments that have only a heavy chain variable region and a light chain variable region are disclosed in PCT International Public Patent Applications WO 88/10649, WO 88/106630, WO 88/07085, WO 88/07086, and WO 88/09344. Two-chain Fvs have a heavy chain variable region and a light chain variable region linked by non-covalent bonds, and single-chain Fvs have a heavy chain variable region and a light chain variable region linked by covalent bonds, typically via a peptide linker, or directly at the C-terminus, which can form a dimer-like structure like double-chain Fvs. These antibody fragments can be obtained using proteinases (e.g., restriction digestion of the whole antibody with papain yields Fab and digestion with pepsin yields the F(ab')2 fragment), or they can be produced by genetic recombination techniques.

As used herein, the term "heavy chain" refers to both full-length heavy chains and fragments thereof comprising a variable region domain VH and three invariant domains C(H1), C(H2), and C(H3) comprising an amino acid sequence having sufficient variable region sequence to confer specificity to an antigen.

As used herein, the term "complementarity determining region (CDR)" refers to the amino acid sequences of the hypervariable regions of the heavy and light chains of immunoglobulins (Kabat et al. Sequences of Proteins of Immunological Interest, 4th Ed., U.S. Department of Health and Human Services, National Institutes of Health (1987)). The heavy chain (HCDR1, HCDR2, and HCDR3) and light chain (LCDR1, LCDR2, and LCDR3) each contain three CDRs, which provide the primary contact residues for antibody binding to an antigen or epitope.

The scope of the antibodies or antibody fragments of the present invention includes variants having conservative amino acid substitutions in the CDR region. Furthermore, the antibodies or antibody fragments of the invention may include variants of the amino acid sequences listed in the attached sequence list, to the extent that they are capable of specifically recognizing phosphorylated PLCγ2. For example, additional changes can be made to the amino acid sequence of the antibody to further improve the binding affinity and/or other biological properties of the antibody. Such modifications include, for example, deletions, insertions, and/or substitutions of amino acid sequence residues of the antibody. These modifications of amino acid are made based on the relative similarity of the amino acid side chain substituents, e.g., hydrophobicity, hydrophilicity, charge, size, etc. By analyzing the size, shape, and type of amino acid side chain substituents, it can be seen that arginine, lysine, and histidine are all positively charged residues; alanine, glycine, and serine have similar sizes; and phenylalanine, tryptophan and tyrosine have similar shapes. Therefore, based on these considerations, arginine, lysine, and histidine; alanine, glycine, and serine; and phenylalanine, tryptophan, and tyrosine are biologically functional equivalents.

In introducing variation, the hydropathic index of an amino acid can be taken into account. Each amino acid is assigned a hydropathic index based on its hydrophobicity and charge: Isoleucine (+4.5); Valine (+4.2); Leucine (+3.8); Phenylalanine (+2.8); Cysteine/Cysteine (+2.5); Methionine (+1.9); Alanine (+1.8); Glycine (-0.4); Threonine (-0.7); Serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

Hydrophobic amino acid indices are crucial in conferring interactive biological functions to proteins. It is well known in the art that substitutions of amino acids with similar hydrophobic indices should have similar biological activity. When introducing variation with reference to hydrophobic index, substitutions are made between amino acids that exhibit a difference in hydrophobic index that is within ± 2 in one particular example, within ± 1 in another particular example, and within ± 0.5 in yet another particular example.

It is also well known that substitutions between amino acids with similar hydrophilicity values result in proteins with equal biological activity. As disclosed in U.S. Patent No. 4,554,101, the following hydrophilicity values are assigned to each of the amino acid residues Arginine (+3.0); Lysine (+3.0); Aspartate (+3.0± 1); Glutamate (+3.0±1); Serine (+0.3); Asparagine (+0.2); Glutamine (+0.2); Glycine (0); Threonine (-0.4); Proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). Where variation is introduced by reference to hydrophilicity values, substitutions are made between amino acids that result in a difference in hydrophilicity values that is within ± 2 in one particular example, within ± 1 in another particular example, and within ± 0.5 in yet another particular example.

Amino acid exchanges in proteins that do not alter the activity of the molecule as a whole are well-known in the art (H. Neurath, R.L. Hill, The Proteins, Academic Press, New York, 1979). The most common exchanges are between the amino acid residues Ala/Ser, Val/Ile, Asp/Glu, Thr/Ser, Ala/Gly, Ala/Thr, Ser/Asn, Ala/Val, Ser/Gly, Thr/Phe, Ala/Pro, Lys/Arg, Asp/Asn, Leu/Ile, Leu/Val, Ala/Glu, and Asp/Gly.

In considering the variants with biologically equivalent activity described above, the antibodies of the invention or the nucleic acid molecules encoding them are interpreted to include sequences that exhibit substantial identity to the sequences listed in the sequence list. Substantial identity means a sequence that, when aligned as closely as possible with any other sequence of the present invention, and when the aligned sequence is analyzed using algorithms of ordinary skill in the art, exhibits at least 61% homology, in one particular example 70% homology, in another particular example 80% homology, and in yet another particular example 90% homology. Alignment methods for sequence comparison are known in the art. Various methods and algorithms for alignments are described in Smith and Waterman, Adv. Appl. Math. (1981) 2:482 Needleman and Wunsch, J. Mol. Bio. (1970) 48:443; Pearson and Lipman, Methods in Mol. Biol. (1988) 24:307-31; Higgins and Sharp, Gene (1988) 73:237-44; Higgins and Sharp, CABIOS (1989) 5:151-3; Corpet et al. Nuc. Acids Res. (1988) 16:10881-90; Huang et al. Comp. Appl. BioSci. (1992) 8:155-65 and Pearson et al. Meth. Mol. Biol. (1994) 24:307-31. The NCBI Basic Local Alignment Search Tool (BLAST) (Altschul et al. J. Mol. Biol. (1990) 215:403-10) is accessible from NBCI and elsewhere, and is available on the Internet in conjunction with sequence analysis programs such as blastp, blasm, blastx, tblastn, and tblastx. BLAST can be accessed at www.ncbi.nlm.nih.gov/BLAST/. Instructions for comparing sequence homology using this program can be found at www.ncbi.nlm.nih.gov/BLAST/blast_help.html.

As used herein, the term "Asporin protein" refers to a protein that in humans is encoded by the ASPN gene and belongs to the leucine-rich repeat (LRR) family of proteins associated with the cartilage matrix. Asporin protein may also be referred to as OS3, PLAP-1, PLAP1, or SLRR1C.

According to a specific embodiment of the invention, the heavy chain variable region of an antibody of the invention or an antigen-binding fragment thereof has the amino acid sequence of SEQ ID NO: 11.

According to a specific embodiment of the invention, the antibody of the invention or an antigen-binding fragment thereof further comprises a light chain variable region having a light chain CDR amino acid sequence of LCDR1 comprising the sequence of SEQ ID NO: 4, LCDR2 comprising the sequence of SEQ ID NO: 5 and LCDR3 comprising the sequence of SEQ ID NO: 6.

As used herein, the term "light chain" refers to both full-length light chains and fragments thereof comprising a variable region domain V_{L} and an invariable region domain C_{L} comprising an amino acid sequence having sufficient variable region sequence to confer specificity to an antigen.

According to a specific embodiment of the invention, the light chain variable region of an antibody of the invention or an antigen-binding fragment thereof has an amino acid sequence of SEQ ID NO: 16.

According to specific embodiments of the present invention, the antigen-binding fragments of the present invention are Fab fragments, F(ab') fragments, F(ab')2 fragments, or Fv fragments.

The antibodies of the present invention include, but are not limited to, monoclonal antibodies, human antibodies, humanized antibodies, chimeric antibodies, short-chain Fvs (scFVs), short-chain antibodies, Fab fragments, F(ab') fragments, disulfide-bound Fvs (sdFVs), and anti-idiotype (anti-Id) antibodies, and epitope-bound fragments of the foregoing.

According to a specific embodiment of the present invention, the antibodies of the present invention are monoclonal antibodies.

As used herein, the term "monoclonal antibody" means an antibody molecule of a single molecular composition obtained from a substantially identical antibody population, wherein the monoclonal antibody exhibits a single binding specificity and affinity for a specific epitope.

According to another aspect of the present invention, the present invention provides a composition for the prevention or treatment of fibrotic disease comprising an antibody of the invention or an antigen-binding fragment thereof as an active ingredient.

As used herein, the term "prevention" means the suppression of the development of a condition or disease in a subject who has not been diagnosed as having the condition or disease, but who is at risk of developing the condition or disease.

As used herein, the term "treatment" means (a) inhibiting the development of a condition, disease, or symptom; (b) alleviating a condition, disease, or symptom; or (c) eliminating a condition, disease, or symptom. When administered to a subject, the compositions of the present invention inhibit the activity of the fibrosis-causing Asporin protein, thereby preventing excessive fibrosis, thereby inhibiting the development of, eliminating or alleviating the symptoms caused by fibrotic disease. Accordingly, the compositions of the present invention may be compositions for the treatment of these diseases by themselves, or may be administered in combination with other pharmacological agents as an adjunct to the treatment of these diseases. Accordingly, as used herein, the term "treatment" or "therapeutic" includes the meanings of "therapeutic aid" or "therapeutic adjunct".

As used herein, the term "fibrotic disease" refers to any pathological condition that involves the progression of pathological tissue fibrosis and is directly or indirectly caused by such fibrosis. The term "fibrosis or fibrosis" also refers to a condition in which there is an excessive accumulation of fibrous connective tissue (a component of the extracellular matrix such as collagen and fibronectin) in and around inflamed or damaged tissue, which can result in permanent scarring, organ dysfunction, and ultimately death.

According to a specific embodiment of the present invention, said fibrotic disease is selected from the group comprising liver fibrosis; kidney fibrosis and pulmonary fibrosis.

As used herein, the term "liver fibrosis" refers to the proliferation of fibrous tissue as a result of chronic damage to the liver, which can be caused by a variety of causes, including biliary fibrosis, post-necrotic scarring, and diffuse liver fibrosis.

As used herein, the term "renal fibrosis" refers to a condition characterized by tubulointerstitial fibrosis and glomerulosclerosis, which is the final manifestation of chronic kidney disease, wherein renal fibrosis is characterized by excessive accumulation and deposition of extracellular matrix components.

As used herein, the term "pulmonary fibrosis" refers to a condition in which the lungs become fibrotic due to repeated damage and injury to the lungs or lung tissue, and is characterized by symptoms of shortness of breath, dry cough, fatigue, weight loss, and nail clubbing.

According to a specific embodiment of the present invention, the pulmonary fibrosis of the present invention is Idiopathic Pulmonary Fibrosis (IPF).

As used herein, the term "idiopathic pulmonary fibrosis" refers to a progressive respiratory disease characterized by thickening and stiffening of lung tissue leading to the formation of scar tissue, and is a type of chronic scarring lung disease characterized by a progressive and irreversible decline in lung function, referred to as "idiopathic" because no definitive cause is known.

According to another aspect of the present invention, the present invention provides a nucleic acid molecule encoding an antibody of the invention or an antigen-binding fragment thereof.

As used herein, the term "nucleic acid molecule" is intended to encompass DNA (gDNA and cDNA) and RNA molecules, and the nucleotide, the basic building block of a nucleic acid molecule, includes not only naturally occurring nucleotides, but also analogs with sugar or base site modifications (Scheit, Nucleotide Analogs, John Wiley, New York

(1980); Uhlman and Peyman, Chemical Reviews, (1990) 90:543-584). The sequences of the nucleic acid molecules encoding the heavy and light chain variable regions of the present invention may be modified. Said modifications comprise additions, deletions, or non-conservative or conservative substitutions of nucleotides.

A nucleic acid molecule of the present invention is interpreted to comprise a nucleotide sequence that exhibits substantial identity to any of the above nucleotide sequences. Substantial identity means a nucleotide sequence that exhibits at least 80% homology, in one particular embodiment at least 90% homology, in another particular embodiment at least 95% homology, and in another particular embodiment at least 95% homology when the nucleotide sequence of the present invention is aligned with any other sequence to the greatest extent possible and the aligned sequence is analyzed using algorithms of ordinary skill in the art.

According to another aspect of the present invention, the present invention provides a composition for the diagnosis of fibrotic disease comprising an antibody of the invention or an antigen-binding fragment thereof as an active ingredient.

According to the present invention, the Asporin protein of the invention can be detected by an immunoassay method using an antigen-antibody reaction and used to analyze the presence of fibrotic disease. Such immunoassays can be performed according to various immunoassay or immunostaining protocols developed in the art.

For example, when the methods of the invention are carried out according to radioimmunoassay methods, antibodies labeled with radioisotopes (e.g., C¹⁴, I¹²⁵, P³², and S³⁵) may be used. In the present invention, the antibodies that specifically recognize the Asporin protein are either polyclonal or monoclonal antibodies, preferably monoclonal antibodies.

The antibodies of the present invention can be prepared by methods conventionally practiced in the art, such as the fusion method (Kohler and Milstein, European Journal of Immunology, 6:511-519 (1976)), the recombinant DNA method (US Patent No. 4,816,567), or the phage antibody library method (Clackson et al, Nature, 352:624-628 (1991) and Marks et al, J. Mol. Biol., 222:58, 1-597 (1991)). A general procedure for the preparation of antibodies is described in Harlow, E. and Lane, D., Using Antibodies: A Laboratory Manual, Cold Spring Harbor Press, New York (1999).

By analyzing the final signal intensity of the immunoassay process described above, it is possible to predict whether the tumor has metastasized or is likely to metastasize. In other words, if the signal for the Asporin protein is stronger in an individual's sample compared to a normal sample, it is likely the individual has metastasized or is likely to metastasize in the future.

As used herein, the term "diagnosis" includes a determination of an entity's susceptibility to a particular disease, a determination of whether an entity currently has a particular disease, and a determination of the prognosis of an entity with a particular disease.

As used herein, the term "diagnostic composition" means a mixture or device comprising a means for measuring the expression of an Asporin protein or a gene encoding an Asporin protein to determine whether a subject has, or is likely to develop, a fibrotic disease, and may also be expressed as a "diagnostic kit".

According to another aspect of the invention, the present invention provides an antibody or antigen-binding fragment thereof directed against an Asporin protein comprising a light chain variable region having a light chain complementarity determining region (LCDR) amino acid sequence of LCDR1 having the sequence of SEQ ID NO: 4, LCDR2 having the sequence of SEQ ID NO: 5, and LCDR3 having the sequence of SEQ ID NO: 6.

Light chain CDRs (LCDRs); light chain variable regions; and antibodies or antigen-binding fragments thereof as used in the present invention have already been described above and are hereby omitted to avoid undue redundancy.

According to another aspect of the invention, the invention provides a method of preventing or treating a fibrotic disease comprising the step of administering to a subject an antibody to an Asporin protein of the invention, or an antigen-binding fragment thereof; or a composition comprising said antibody or antigen-binding fragment thereof as an active ingredient.

Antibodies to the Asporin protein of the present invention or antigen-binding fragments thereof; compositions comprising the same as active ingredients; and fibrotic diseases that can be prevented or treated with the same have already been described above and are hereby omitted to avoid undue repetition.

### Advantageous Effects

The features and benefits of the present invention are summarized below:
(a) The present invention provides methods for predicting the development of a fibrotic disease using an antibody that specifically binds to an Asporin protein involved in fibrosis, or for preventing or treating a fibrotic disease by inhibiting the activity of the protein.
(b) The present invention provides effective antibodies that can be used for the diagnosis, prevention and treatment of idiopathic pulmonary fibrosis, a disease for which no clear pathogenesis is known and for which efficient treatment methods have been lacking.
(c) Furthermore, the present invention may ultimately be useful for efficiently blocking fibrosis in a variety of tissues by providing a fundamental inhibition method for pathological fibrosis mechanisms.

### Brief Description of Drawings

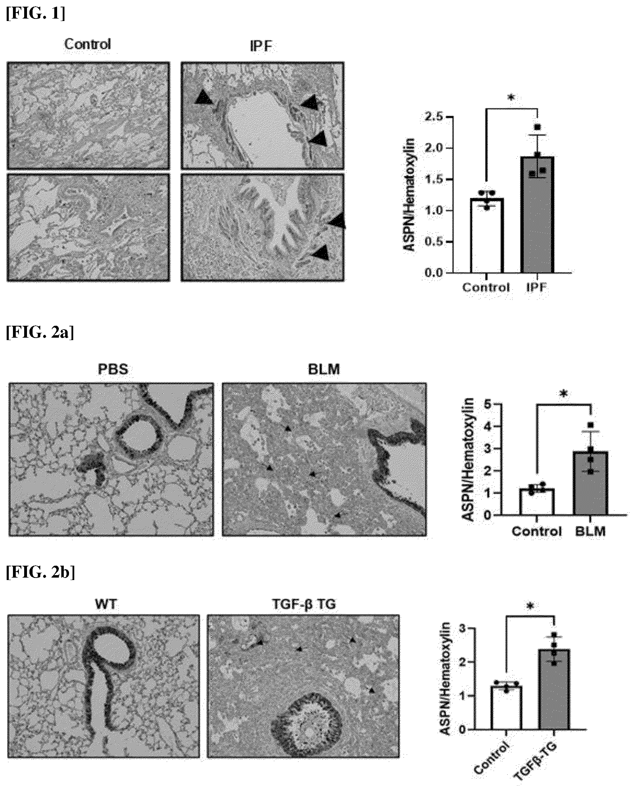

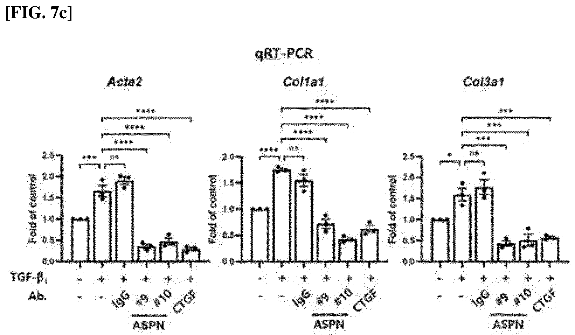

FIG. 1a is an illustration of immunohistochemical staining results of Human Lung Tissue, wherein the left side shows staining results in control and IPF patients, and the right side is a graph showing a quantitative comparison of Asporin expression in control and IPF patients, with Asporin expression on the y-axis.
FIG. 2a is an immunohistochemical staining for Asporin (left) and a graph showing expression (right) in a mouse lung fibrosis model of bleomycin-induced fibrosis. FIG. 2b is a graph showing immunohistochemical staining for Asporin (left) and expression of Asporin (right) in a mouse lung fibrosis model with TGF-β transgenic lung fibrosis.

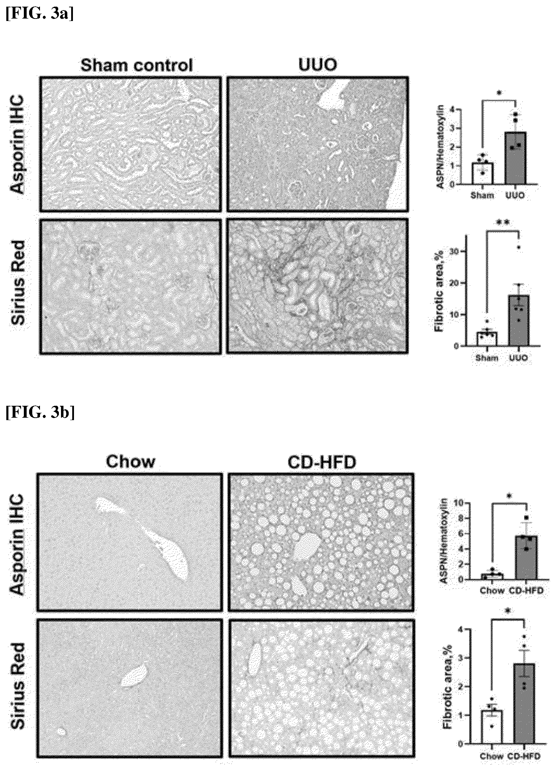

FIG. 3a shows immunohistochemical staining for Asporin in a mouse model of kidney fibrosis, Unilateral Ureter Obstruction (UUO) (top left), collagen observation using Sirius Red staining (bottom left), a graph showing Asporin expression (top right), and a graph showing fibrosis area measurement using Sirius Red staining (bottom right). FIG. 3b shows immunohistochemical staining for Asparin in a mouse model of liver fibrosis, choline-deficient high fat diet (CD-HFD) mice (top left), collagen observation using Sirius Red staining (bottom left), a graph showing Asparin expression (top right), and a graph showing fibrosis area measurement using Sirius Red staining (bottom right).

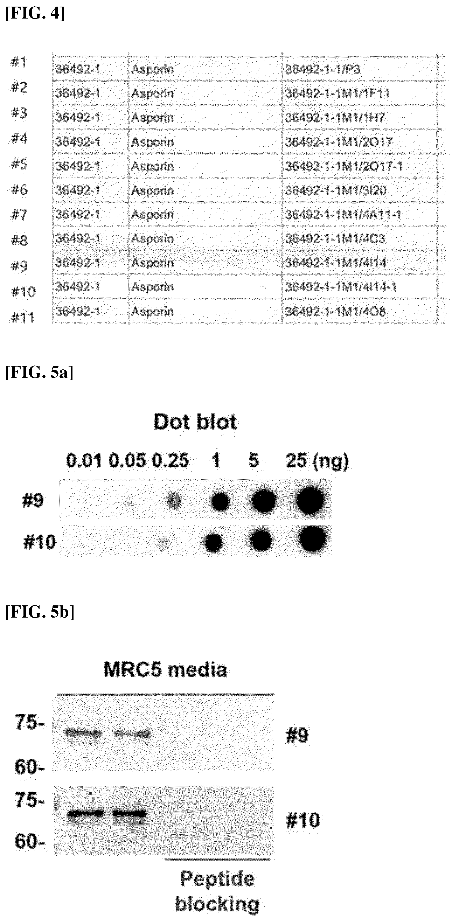

FIG. 4 is a table depicting monoclonal antibodies #1 through #11 produced for the development of therapeutics targeting Asporin.
FIG. 5a shows the results of dot blotting performed on two clones with good affinity. FIG. 5b shows the results of peptide blocking performed on MRC5, a lung fibroblast cell.

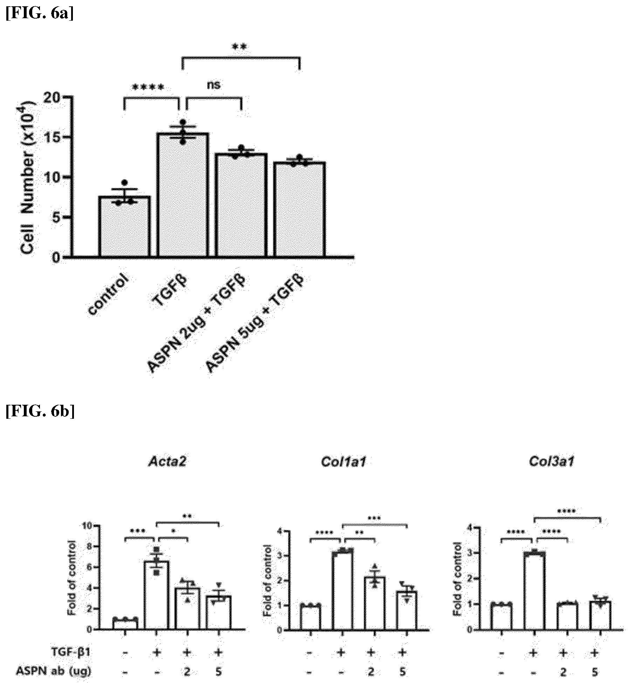

FIG. 6a is a graph showing that TGF-βdp-induced cell proliferation in human fibroblasts is reduced upon treatment with the Asporin antibody. FIG. 6b is a graph showing that the target genes of TGF-βdp-induced fibrosis (*Acta 2, Colla1* and *Col3a1*) in human fibroblasts were reduced by Asporin antibody treatment.

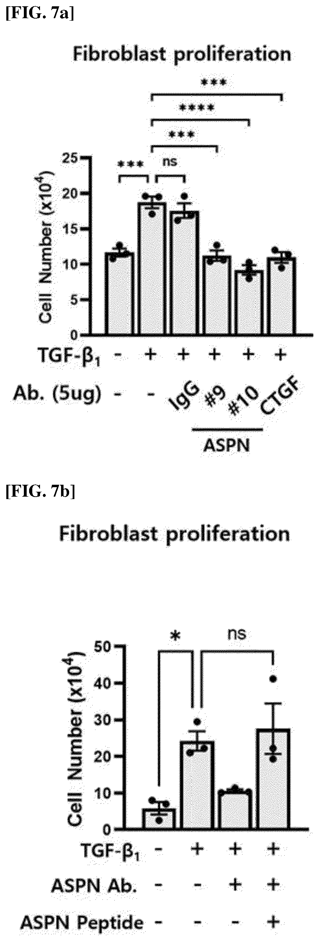

FIG. 7a is a graph showing the results of comparing the fibroblast proliferation inhibitory activity of the anti-A
Asporin antibodies (#9 and #10) of the present invention with a CTGF antibody (CTGF Ab.). FIG. 7b is a graph showing the results of verifying the specificity of the antibodies by measuring the degree of peptide blocking through the fibroblast proliferation inhibition ability of the present invention. FIG. 7c is a graph showing the results of validating the fibrosis target genes (*Acta2, Colla1, Col3a1*) of the anti-Asporin antibodies (#9 and #10) of the present invention using qRT-PCR.

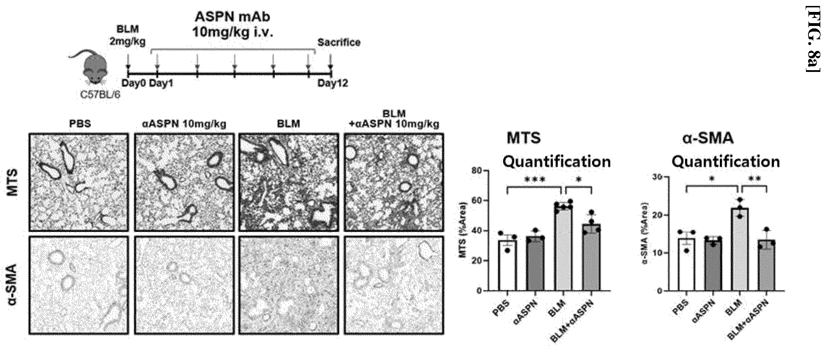

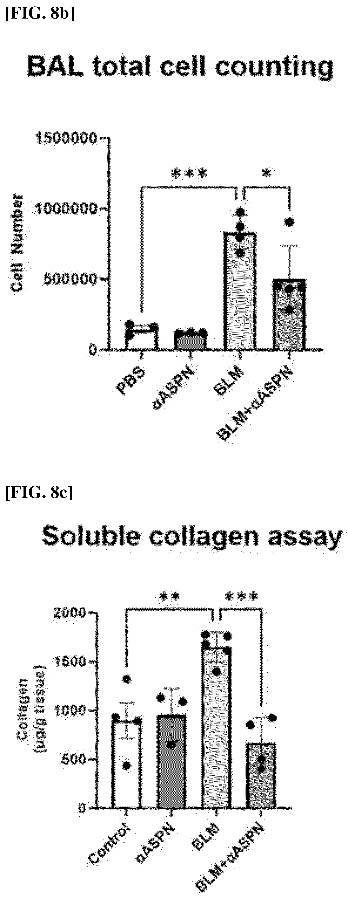

FIG. 8a is a graph illustrating the appearance of fibrosis in tissue following administration of the antibody (#10) of the present invention in a mouse model of pulmonary fibrosis and the quantification of fibrosis by the MTS alc α-SMA method. FIG. 8b is an illustration of the results of counting cells in bronchoalveolar lavage fluid collected from a mouse model of pulmonary fibrosis. FIG. 8c is a graph depicting the results of quantification of soluble collagen in a mouse model of pulmonary fibrosis.

### Mode for Invention

Hereinafter, the present invention will be described in further detail by examples. It would be obvious to those skilled in the art that these examples are intended to be more concretely illustrative and that the scope of the present invention as set forth in the appended claims is not limited to or by the examples.

### Example

### Experimental Methods and Analysis

### Animal models

The mice were housed in a specific pathogen-free animal facility, with a controlled temperature and humidity under conditions of a 12-h light/12-h dark cycle. Before administration of BLM, 8-week-old mice were injected with 10 mg/kg tamoxifen (Merck, Darmstadt, Germany) three times per week for 1 week. Mice were anesthetized with an intraperitoneal injection of Zoletil 50 (30 mg/kg) and Rompun (10 mg/kg). Mice were intratracheally injected with PBS (vehicle control) or 4 mg/kg BLM (Santa Cruz Biotechnology, Dallas, TX, USA). An average of eight mice were used in each group.

### Immunostaining

In preparation for IHC, lung section slides were rehydrated through a graded alcohol series, followed by antigen retrieval in 10 mM sodium citrate. The IHC staining protocol included the sequential application of a peroxidase-blocking reagent, primary antibody (O/N), secondary antibody (1 hour) molecules and horseradish peroxidase coupled to a polymer backbone, and visualization using a 3,3'-diaminobenzidine tetrahydrochloride (DAB) chromogen reagent with a hydrogen peroxide substrate (Dako, Santa Clara, CA, USA). Intensity of Asporin protein was calculated using ImageJ. In addition, the Asporin antibody was Thermo PA5-28124.

### Operation of Unilateral Ureter Obstruction (UUO) mouse fibrosis model

Mechanical stress was applied to induce renal fibrosis in mice. Mice were anesthetized with an intraperitoneal injection of Zoletil (50mg/ml) and Rompun (23.32mg/ml), and incisions were made in the skin and peritoneum with forceps and a medical Iris Scissor. One kidney was identified, the ureter was severed with a non-pointed hook, and the ureter was tied twice with black silk thread. The peritoneum and skin were then sutured with black silk thread and sterilized with povidone-iodine. The mice were sacrificed on day 8 after unilateral ureteral obstruction surgery.

### Prepare Kidney Tissue Sections for histological Observation

Kidney with no-capsule, were instilled with 4% formaldehyde and fixed under pressure for 24 h at 4 °C . After tissue processing, embedded in paraffin blocks and sectioned at 4 µm.

### Sirius red staining for observation collagen

Using FFPE kidney tissue, Remove paraffin with xylene and Hydration with 100%,90%, 80%, EtOH. After hydration, kidney tissue were instilled with picro Sirius red (ab150681, Abcam, Cambridge, UK) for 1hour and washing with 0.5% acidified water twice. Mounting the tissue with hydrophobic mounting solution.

### Choline-deficient High Fat Diet (CD-HFD) mouse model

To create the CD-HFD model, we refer to Int J Exp Pathol. Apr;94(2):93-103. (2013). In addition, CDAA, HFD with 0.1% methionine (CDAHFD; #A06071302) were purchased from Research Diets (New Brunswick, NJ, USA). These HFDs provided 5.2 kcal per gram and contained 320 g/kg of lard-based fat for 8 weeks.

### Dot blot

Dot blotting was performed using the steps below.
(1) Delivery antibody quality control: dot blot detection of corresponding peptide antigens
(2) BSA conjugated were attached to nitrocellulose membranes (each point was 2 µL). Groups of points contained, from right to left, 25 ng, 5 ng, 1 ng, 0,25 ng, 0.05 ng, and 0.01 ng of conjugated polypeptide (FIG. 5a). The blots were blocked using 5% skim milk/TBST.
(3) After incubation with 1:1000 diluted antibody for 1 hour, washed 3 times with TBST, and incubated with secondary antibody for 45 minutes.
(4) After adding ECL reagent and reacting for 1 minute, preservative film was added and the X-ray film was exposed and developed in the dark (several exposures were made).

### Peptide Blocking and Western Blotting

Cells were lysed in lysis buffer (20 mM Tris-Cl, 150 mM NaCl, 1% Triton X-100, 1.5% MgCl₂, 1 mM EDTA, 1 mM Na2VO4, 1 mM phenylmethylsulfonyl fluoride (PMSF) and protease inhibitor cocktail, pH 7.5). Lysates were briefly vortexed and sonicated and cleared by centrifugation at 12,000 x g for 20 minutes at 4 °C . The supernatants were collected and transferred to fresh tubes. Protein concentrations were determined by 660 nm protein assay reagent (Thermo Scientific, Waltham, MA, USA). Equal amount of protein extracts was subjected to electrophoresis on SDS-polyacrylamide gels and then transferred to Nitrocellulose transfer membranes (Whatman, Dassel, Germany). The membranes were blocked in PBS containing 0.1% (v/v) Tween 20 (Sigma-Aldrich, St. Louis, MO, USA) and 5% (w/v) nonfat DifcoTM skim milk (BD Biosciences, Sparks, MD, USA) and 3% BSA (Affymetrix, Santa clara OH, USA) and probed with primary antibodies. The following antibodies were used: anti-Asporin (Abmart, China) The membranes were then washed with 1x PBST, incubated with the appropriate secondary anti-mouse horseradish peroxidase-conjugated antibodies (Thermo Scientific, Rockford IL, USA) for 1 hour, and visualized using the LAS-3000 system (Fujifilm, Stamford, CT, USA) with an enhanced chemiluminescence detection reagent (Thermo Scientific).

For peptide blocking, diluted primary antibody was incubated with peptides of ASPN sequence for 4 hours. It was used for blotted membrane incubation. Furthermore, the sequence of the Asporin peptide is as follows: CPFGCQCYSRVVVHCSDLGLT (SEQ ID NO: 23).

### Media concentration

Medium from MRC5 cells cultured in 60-mm cell culture dishes was harvested, passed through a 0.45 µm filter (Sartorius, Stonehouse, UK), and used as conditioned medium. Additionally, culture medium was applied to a VIVASPIN6 column (Sartorius) and centrifuged for 2 hours at 10,000 x g; concentrated protein was calculated using a protein assay, and then immunoblotted using antibodies.

### Cell culture

Human fibroblast cell line MRC5 were purchased from Korean Cell Lines Bank (Seoul, Korea). Cells were cultured in Dulbecco's modified Eagle's medium supplemented with 10% (v/v) fetal bovine serum and 1% antibiotic/antimycotic solution (Corning, Manassas, VA, USA) at 37 °C under 5% CO₂.

### MRC5 Human Fibroblast cell Proliferation

The cells were serum-starved for 2 hours before TGF-β1 (20 ng/ml) and monoclonal antibody treatment for 24 hours. Cells were trypsinized and counted on a flow cytometer.

### RNA isolation and quantitative reverse transcription polymerase chain reaction (qRT-PCR)

Total RNA was extracted using TRIzol reagent following the manufacturer's protocol (Takara, Kyoto, Japan). The 500 µl of Trizol was added to cells culture dishes (6 well) and cells were collected to tube. The 100 µl of chloroform was added to samples and the samples were vortexed. The samples were incubated for 3 minutes at room temperature and centrifuged at 12,000 x g for 15 minutes at 4 °C . The 250 ul of supernatant was collected and transferred to fresh tube. The 250 µl of isopropanol was added to samples and samples were centrifuged at 12,000 x g for 10 minutes at 4 °C . The supernatant was removed and the 500 µl of 75% ethanol was added to samples. The sample was centrifuged at 13,000 x g for 5 minutes at 4 °C . The supernatant was removed and pellet was dried at room temperature. The 30 µl of DEPC was added to dried pellet. The concentration of RNA was measured by Nanodrop 1000 (Thermo scientific, Waltham, MA, USA). After RNA isolation, the 1 µg of total RNA was used and cDNA was synthesized using CellScript (CellSafe, Seoul, Korea), following to the manufacturer's protocol.

qRT-PCR analyses were performed using the ABI PRISM 7000 Sequence Detection System instrumentation and software (Applied Biosystems, Carlsbad, CA, USA) according to the manufacture's protocol with minor modification. Briefly, the appropriate amount of the reverse transcription reaction mixture was amplified with specific primers using SYBR green PCR master mix (Applied Biosystems, Carlsbad, CA, USA). PCR primer sequences for qRT-PCR are listed in Table 1.

**[Table 1]**

| Targets | Sense | Antisense |
|---|---|---|
| human COL1a1 | CCTCAAGGGCTCCAAC (SEQ ID NO: 33) | GGTTTTGTATTCAATCACTG TCTTGC (SEQ ID NO: 34) |
| human COL3a1 | TGGTCTGCAAGGAATGCCTG GA (SEQ ID NO: 35) | TCTTTCCCTGGGACACCATC AG (SEQ ID NO: 36) |
| human α-SMA | CTGGCATCGTGCTGGACTCT (SEQ ID NO: 37) | GATCTCGGCCAGCCAGATC (SEQ ID NO: 38) |

### Analyze statistics

Results were visualized and analyzed with Prism software, version 5 (GraphPad Software, San Diego, CA, USA). When more than two groups of samples were compared, one-way ANOVA was examined for statistical significance. Data values were shown as the mean±standard deviations (SD). Student's t-test used for determining statistical significance of two groups. Values of P < 0.05 were considered statistically significant.

### Experimental results

### In IPF patients, asporin protein expression was found to be increased

The above immunohistochemical staining of lung section slides from IPF patients revealed increased expression of the protein Asporin in IPF patients. The left side of FIG. 1 shows immunohistochemical staining results from control and IPF patients, with arrows indicating areas of histochemical staining due to asporin expression in IPF patients. The right side of FIG. 1 is a graph showing the expression of ASPN (y-axis) in control and IPF patients. The results show that the expression of Asporin is significantly increased in IPF patients compared to controls.

### Asporin expression is significantly increased in mouse models of pulmonary fibrosis

Since bleomycin has side effects of lung injury and lung fibrosis, we used it to obtain a mouse model with advanced lung fibrosis. We also prepared a mouse model in which lung fibrosis was induced by transgenic transfection of the TGF-β gene. Immunohistochemical staining of mouse lung tissue from these lung fibrosis-induced mouse models showed that the expression of Asporin was significantly increased in all of them.

### Significantly increased Asporin expression in a mouse model of renal fibrosis and a mouse model of liver fibrosis

In the Unilateral Ureter Obstruction (UUO) mouse model, a model of kidney fibrosis, and the choline-deficient high fat diet (CD-HFD) mouse model, a model of liver fibrosis, we found that the expression of Asporin was significantly increased upon induction of fibrosis. FIG. 3a shows the experimental results for UUO and FIG. 3b shows the experimental results for CD-HFD, and graphs of immunohistochemical staining results and Asporin expression and fibrosis area are shown for each model. From these results, it is clear that Asporin is increased by fibrosis in the kidney and liver.

### Affinity of monoclonal antibody candidates to Asporin was investigated to select antibodies with high specificity

Clones of the anti-Asporin antibodies of #1 to #11 were obtained as shown in FIG. 4. Of these, two clones with high specificity were investigated and screened by dot blotting and peptide blocking, and one of them (#10) was confirmed to have an inhibitory effect on fibrosis.

### Confirmed the inhibitory effect of selected monoclonal antibodies against fibrosis

We experimentally confirmed that the selected monoclonal antibody (#10) inhibited fibroblast proliferation and inhibited fibrosis target gene expression, thus confirming the inhibitory effect of the selected monoclonal antibody on fibrosis and its preventive or therapeutic effect on fibrotic diseases.

### Confirmed anti-fibrotic activity of selected antibodies in cellular models

We found that TGF-β-induced cell proliferation in human fibroblasts was reduced upon Asporin antibody treatment. Furthermore, TGF-β induced fibrosis target genes in human fibroblasts were reduced after treatment with Asporin antibody. Thus, the anti-fibrosis efficacy of the anti-Asporin antibodies of the present invention was finally confirmed.

*The CDR sequences of the selected antibodies are as follows*
HCDR1 : GFTFSDYW (SEQ ID NO: 1)
HCDR2 : IRNKPYNYETY (SEQ ID NO: 2)
HCDR3 : TAYYRYDVLFDY (SEQ ID NO: 3)
LCDR1 : QDISNY (SEQ ID NO: 4)
LCDR2 : YTS (SEQ ID NO: 5)
LCDR3 : QQSKTLPLT (SEQ ID NO: 6)
HFR1 : EVKLVETGGGLVQPGRPMKLSCVAS (SEQ ID NO: 7)
HFR2 : MNWVRQSPEKGLEWVVQ (SEQ ID NO: 8)
HFR3 : YYSDSVKGRFTISRDDSKSCVYLQMNNLRAEDMGIYYC (SEQ ID NO: 9)
HFR4 : WGQGTSLTVSS (SEQ ID NO: 10)
Heavy chain variable region: 
EVKLVETGGGLVQPGRPMKLSCVASGFTFSDYWMNWVRQSPEKGLEWVVQIR
NKPYNYETYYSDSVKGRFTISRDDSKSCVYLQMNNLRAEDMGIYYCTAYYRYD
VLFDYWGQGTSLTVSS (SEQ ID NO: 11)
LFR1 : DIQMTQTTSSLSASLGDRVTISCRAS (SEQ ID NO: 12)
LFR2 : LNWYQQKPEGTVKLLIY (SEQ ID NO: 13)
LFR3 : RLHSGVPSRFSGSGSGTDYSLTISNLEQEDIATYFC (SEQ ID NO: 14)
LFR4 : FGAGTKLELK (SEQ ID NO: 15)
Light chain variable region:

DIQMTQTTSSLSASLGDRVTISCRASQDISNYLNWYQQKPEGTVKLLIYYTSRLH
SGVPSRFSGSGSGTDYSLTISNLEQEDIATYFCQQSKTLPLTFGAGTKLELK (SEQ
ID NO: 16)

### Nucleic acid sequences

HCDR1 : ggattcacttttagtgactactgg (SEQ ID NO: 17)
HCDR2 : attagaaacaaaccttataattatgaaaca (SEQ ID NO: 18)
HCDR3 : acagcctactataggtacgacgtcctcttcgactac (SEQ ID NO: 19)
LCDR1 : caggacattagcaattat (SEQ ID NO: 20)
LCDR2 : tacacatca (SEQ ID NO: 21)
LCDR3 : caacagagtaaaacgcttcctctcacg (SEQ ID NO: 22)

HFR1:
gaggtgaagctggttgagactggaggaggcttggtgcaacctgggaggcccatgaaactctcctgtgttgcctct (SEQ ID NO: 23)
HFR2: atgaactgggtccgccagtctccagagaaaggactggagtgggtagtacaa (SEQ ID NO: 24)
HFR3: 
tattattcagattctgtgaaaggcagattcaccatctcaagagatgattccaaaagttgtgtctacctgcaaatgaacaacttaaga

gctgaagacatgggtatctattactgt (SEQ ID NO: 25)
HFR4: tggggccaaggcacctctctcacagtctcctcag (SEQ ID NO: 26)
Heavy chain variable region :

gaggtgaagctggttgagactggaggaggcttggtgcaacctgggaggcccatgaaactctcctgtgttgcctctggattcact

tttagtgactactggatgaactgggtccgccagtctccagagaaaggactggagtgggtagtacaaattagaaacaaaccttata

attatgaaacatattattcagattctgtgaaaggcagattcaccatctcaagagatgattccaaaagttgtgtctacctgcaaatgaa

caacttaagagctgaagacatgggtatctattactgtacagcctactataggtacgacgtcctcttcgactactggggccaaggc

acctctctcacagtctcctcag (SEQ ID NO: 27)

LFR1:
gatatccagatgacacagactacatcctccctgtctgcctctctctgggagacagagtcaccatcagttgcagggcaagt (SEQ ID NO: 28)
LFR2 : ttaaactggtatcagcagaaaccagaaggaactgttaaactcctgatctac (SEQ ID NO: 29)
LFR3: 
agattacactcaggagtcccatcaagattcagtggcagtgggtctggaacagattattctctcaccattagcaacctggaaca

agaagatattgccacttacttttgt (SEQ ID NO: 30)
LFR4: ttcggagctgggaccaagttggagctgaaac (SEQ ID NO: 31)
Light chain variable region :

gatatccagatgacacagactacatcctccctgtctgcctctctgggagacagagtcaccatcagttgcagggcaagtcaggac

attagcaattatttaaactggtatcagcagaaaccagaaggaactgttaaactcctgatctactacacatcaagattacactcagg

agtcccatcaagattcagtggcagtgggtctggaacagattattctctcaccattagcaacctggaacaagaagatattgccactt

acttttgtcaacagagtaaaacgcttcctctcacgttcggagctgggaccaagttggagctgaaac (SEQ ID NO: 32)

### valuation of the efficacy of an anti-Asporin antibody in a mouse model of pulmonary fibrosis

To evaluate the efficacy of the #10 anti-Asporin antibody described above, we further confirmed its inhibitory effect on fibrosis in an animal model. The animal model was a mouse model of pulmonary fibrosis.

Pulmonary fibrosis was induced in 8-10 week-old C57BL/6 mice by intrabronchial injection of bleomycin at a dose of 2mg/kg, and mice were treated with Asporin antibody 10mg/kg intravenously every other day for 5 times starting from the next day, and mice were sacrificed on day 12 for experiments. Paraffin sections of mouse lungs were examined for tissue fibrosis using Masson's trichrome staining (MTS) and immunohistochemical staining of α-SMA, and quantified using ImageJ software. It was observed that bleomycin-induced lung fibrosis was reduced upon injection of the anti-Asporin antibody of the present invention (FIG. 8a).

Bronchoalveolar Lavage Fluid was taken from the lungs of mice and cells were counted. It was observed that the bleomycin-induced increase in cell number was significantly reduced in the group treated with the anti-Asporin antibody of the present invention (FIG. 8b).

Furthermore, soluble collagen was measured in the lungs of the mice. When measured using the Sircol collagen assay kit (Biocolor, UK), it was observed that the amount of collagen increased by bleomycin was significantly reduced upon injection of the anti-Asporin antibody of the present invention (FIG. 8c).

In conclusion, the excellent fibrosis inhibitory effect of the anti-Asporin antibody of the present invention was validated using animal models.

While the foregoing has described certain aspects of the present invention in detail, it will be apparent to one of ordinary skill in the art that these specific descriptions are merely preferred embodiments and are not intended to limit the scope of the invention. Accordingly, it will be understood that the substantial scope of the invention is defined by the appended claims and their equivalents.

## Claims

1. An antibody to Asporin protein, or an antigen-binding fragment thereof, comprising a heavy chain variable region having a heavy chain complementarity determining region (CDR) amino acid sequence of HCDR1 having the sequence of SEQ ID NO: 1, HCDR2 having the sequence of SEQ ID NO: 2, and HCDR3 having the sequence of SEQ ID NO: 3.

2. The antibody or antigen-binding fragment thereof of claim 1, wherein the heavy chain variable region comprises the amino acid sequence of SEQ ID NO: 11.

3. The antibody or antigen-binding fragment thereof of claim 1, wherein the antibody or antigen-binding fragment further comprises a light chain variable region having the light chain CDR amino acid sequence of LCDR1 having the sequence of SEQ ID NO:4, LCDR2 having the sequence of SEQ ID NO:5, and LCDR3 having the sequence of SEQ ID NO:6.

4. The antibody or antigen-binding fragment thereof of claim 3, wherein the light chain variable region comprises the amino acid sequence of SEQ ID NO: 16.

5. The antibody or antigen-binding fragment thereof of claim 1, wherein the antigen-binding fragment is a Fab fragment, an F(ab') fragment, an F(ab')2 fragment, or an Fv fragment.

6. The antibody or antigen-binding fragment thereof of claim 1, wherein the antibody is a monoclonal antibody.

7. A composition for preventing or treating fibrotic disease, comprising an antibody or an antigen-binding fragment thereof as an active ingredient, as defined in any one of claims 1 to 6.

8. The composition of claim 7, wherein the fibrotic disease is a disease selected from the group comprising liver fibrosis; kidney fibrosis; and pulmonary fibrosis.

9. The composition of claim 8, wherein the pulmonary fibrosis is idiopathic pulmonary fibrosis (IPF).

10. A nucleic acid molecule encoding the antibody or an antigen-binding fragment thereof, as defined in any one of claims 1 to 6.

11. A composition for diagnosing fibrotic disease, comprising as an active ingredient an antibody or an antigen-binding fragment thereof, as defined in any one of claims 1 to 6.

12. An antibody to Asporin protein, or an antigen-binding fragment thereof, comprising a light chain variable region having the light chain complementarity determining region (CDR) amino acid sequence of LCDR1 having the sequence of SEQ ID NO: 4, LCDR2 having the sequence of SEQ ID NO: 5, and LCDR3 having the sequence of SEQ ID NO: 6.
